(19) Europäisches Patentamt
European Patent Office
Office européen des brevets

(11) Veröffentlichungsnummer: **0 135 838**
**B1**

(12) # EUROPÄISCHE PATENTSCHRIFT

(45) Veröffentlichungstag der Patentschrift: **18.04.90**

(51) Int. Cl.$^5$: **C 07 D 231/14,** A 01 N 43/56

(21) Anmeldenummer: **84110287.4**

(22) Anmeldetag: **29.08.84**

(54) **3-Phenyl-4-methoxycarbonylpyrazole, Verfahren zu ihrer Herstellung und ihre Verwendung zur Bekämpfung unerwünschten Pflanzenwuchses.**

(30) Priorität: **02.09.83 DE 3331692**

(43) Veröffentlichungstag der Anmeldung:
**03.04.85 Patentblatt 85/14**

(45) Bekanntmachung des Hinweises auf die Patenterteilung:
**18.04.90 Patentblatt 90/16**

(84) Benannte Vertragsstaaten:
**AT BE CH DE FR GB IT LI NL**

(56) Entgegenhaltungen:
**EP-A-0 019 760**
**US-A-4 116 673**
**US-A-4 401 821**

(73) Patentinhaber: **BASF Aktiengesellschaft**
**Carl-Bosch-Strasse 38**
**D-6700 Ludwigshafen (DE)**

(72) Erfinder: **Plath, Peter, Dr.**
**Berner Weg 24**
**D-6700 Ludwigshafen (DE)**
Erfinder: **Eicken, Karl, Dr.**
**Am Huettenwingert 12**
**D-6706 Wachenheim (DE)**
Erfinder: **Sauter, Hubert, Dr.**
**Neckarpromenade**
**D-6800 Mannheim 1 (DE)**
Erfinder: **Wuerzer, Bruno, Dr. Dipl.-Landwirt**
**Ruedigerstrasse 13**
**D-6701 Otterstadt (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Anmeldung betrifft 3-Phenyl-4-methoxycarbonyl-pyrazole, Verfahren zur ihrer Herstellung, Herbizide, die diese Verbindungen als Wirkstoffe enthalten, sowie Verfahren zur Bekämpfung unerwünschten Pflanzenwuchses mit diesen Verbindungen.

Es ist bekannt, daß Methylester von geeignet substituierten Pyrazol-4-carbonsäuren als Herbizide verwendet werden können (EP—OS 2180; EP—OS 7990; EP—OS 19 760; US—PS 4 116 673).

Es wurde gefunden, daß 3-Phenyl-4-methoxycarbonyl-pyrazole der Formel

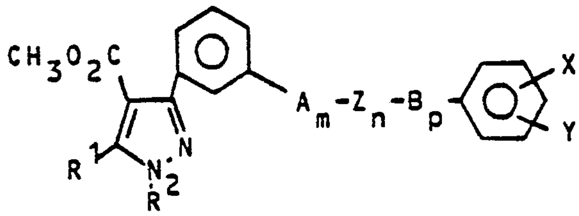 (I),

in der

$R^1$ für Wasserstoff oder Methyl,

$R^2$ für Wasserstoff, gegebenenfalls durch Halogen substituiertes $C_1$—$C_4$-Alkanoyl, Methoxycarbonyl, gegebenenfalls durch Halogen oder $C_1$—$C_4$-Alkyl substituiertes Phenoxycarbonyl, für Hydroxymethyl oder Methoxymethyl,

A und B unabhängig voneinander für Sauerstoff oder Schwefel,

Z für eine gegebenenfalls durch Methyl substituierte Alkylenkette mit 1 bis 5 Kohlenstoffatomen,

m, n und p unabhängig voneinander für die Zahlen Null oder 1,

X für Wasserstoff, Halogen oder Nitro und

Y für Wasserstoff, $C_1$—$C_4$-Alkyl, $C_1$—$C_4$-Alkoxy, $C_1$—$C_4$-Halogenalkyl, Halogen, Nitro oder Cyan stehen,

eine gute herbizide Wirkung haben und gegenüber bestimmten Kulturpflanzen selektiv sind.

Die Pyrazolderivate der Formel I, wobei $R^2$ Wasserstoff bedeutet, können auch als Salze mit üblichen anorganischen oder organischen Säuren wie Chlorwasserstoffsäure, Schwefelsäure, Ameisensäure, Essigsäure, Propionsäure, Trichloressigsäure, Dichlorpropionsäure, Methansulfonsäure oder p-Toluolsulfonsäure vorliegen.

Die Pyrazole der Formel I liegen als Tautomere vor. Die Formeln der Pyrazole umfassen daher stets auch die tautomeren Moleküle.

Die gegebenenfalls substituierte Alkylenkette mit 1 bis 5 Kohlenstoffatomen in Formel I kann beispielsweise Methylen, Ethylen, Methylmethylen, Methylethylen, Propylen, Methylpropylen, Butylen, Methylbutylen, Pentylen oder Methylpentylen bedeuten. Halogen in Formel I bedeutet Fluor, Chlor, Brom, gegebenenfalls durch Halogen substituiertes $C_1$—$C_4$-Alkanoyl kann beispielsweise Formyl, Acetyl, Propionyl, Chloracetyl oder Trifluoracetyl, $C_1$—$C_4$-Alkyl kann beispielsweise Methyl, Ethyl, n-Butyl, insbesondere Methyl, $C_1$—$C_4$-Alkoxy kann beispielsweise Methoxy, Ethoxy, n-Propoxy, insbesondere Methoxy, $C_1$—$C_4$-Halogenalkyl kann beispielsweise Trifluormethyl bedeuten. Mögliche Substituenten für Phenoxycarbonyl sind beispeilsweise Chlor, Brom oder Methyl.

Bevorzugte Pyrazolderivate der Formel I sind solche, bei denen $R^1$ Methyl bedeutet. $R^2$ in Formel I bedeutet vorzugsweise Wasserstoff oder Acetyl.

Man erhält die Pyrazole der Formel I, wobei $R^2$ Wasserstoff bedeutet, in an sich bekannter Weise durch Umsetzung von 3-Alkylamino-propensäure-methylestern der Formel

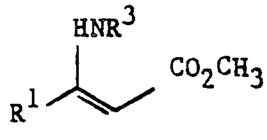 (II),

in der

$R^1$ für Wasserstoff oder Methyl und

$R^3$ für $C_1$—$C_4$-Alkyl stehen,

mit Benzosäurehalogeniden der Formel

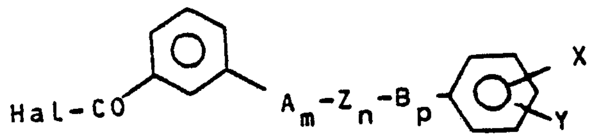

(III),

in der
Hal für Halogen, vorzugsweise für Chlor oder Brom, steht und A, B, Z, X, Y, m, n und p die obengenannten Bedeutungen haben, zu 2-Benzoyl-propensäuremethylestern der Formel

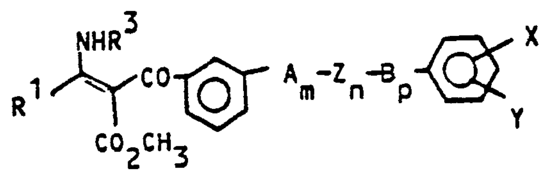

(IV),

in der
$R^1$, $R^3$, A, Z, B, X, Y, m, n und p die obengenannten Bedeutungen haben, und weitere Umsetzung der Ester der Formel IV mit mindestens der äquimolaren Menge Hydrazinhydrat (Synthesis *1982,* S. 318 f).

Pyrazolderivate der Formel I, wobei $R^2$ gegebenenfalls durch Halogen substituiertes $C_1$—$C_4$-Alkanoyl, Methoxycarbonyl oder gegebenenfalls durch Halogen oder $C_1$—$C_4$-Alkyl substituiertes Phenoxycarbonyl bedeuten, lassen sich in an sich bekannter Weise durch Umsetzung mit Acylierungsmitteln, wie Ameisensäure-essigsäure-anhydrid, Actanhydrid, Chlorkohlensäuremethylester, Chlorkohlensäurephenylester oder Chloracetylchlorid, aus den Pyrazolderivaten der Formel I, bei denen $R^2$ für Wasserstoff steht, herstellen.

Die Pyrazole der Formel I, bei denen $R^2$ für Wasserstoff steht, können außerdem nachträglich durch Umsetzung mit Formaldehyd in 1-Stellung des heterocyclischen Fünfrings hydroxymethyliert oder durch Umsetzung mit Chlormethylether methoxymethyliert werden.

Der Reaktionsablauf läßt sich am Beispiel des 5-Methyl-4-methoxycarbonyl-3-(3-benzyloxyphenyl)-pyrazols durch das folgende Reaktionsschema wiedergeben:

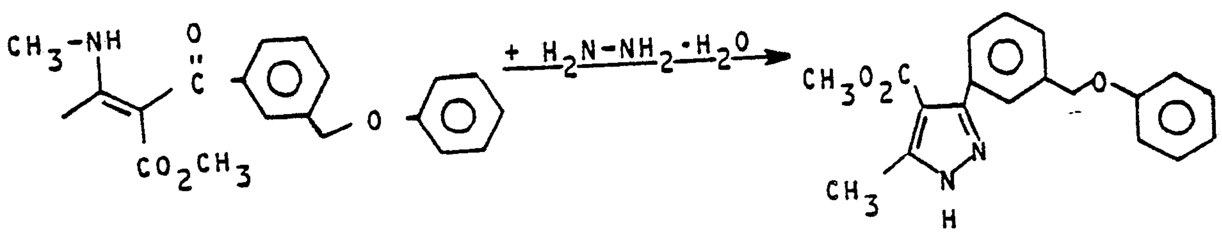


Zur Umsetzung des 2-Benzoyl-propensäure-methylesters der Formel IV mit Hydrazinhydrat wird der Ester in einem Verdünnungsmittel, wie Eisessig, Diethylether, Toluol, Pyridin, Salzsäure oder Wasser, vorzugsweise in Eisessig, gelöst und bei 20 bis 50°C mit mindestens der äquimolaren Menge Hydrazinhydrat versetzt. Zur Vervollständigung der Umsetzung kann es erforderlich sein, die Reaktionsmischung vor der Aufarbeitung bis zu 110°C zu erwärmen.

Zur Gewinnung des Pyrazols der Formel I zeiht man das Verdünnungsmittel gegebenenfalls ab, neutralisiert die Reaktionsmischung mit wäßrigem Ammoniak und extrahiert das Produkt der Formel I mit Extraktionsmitteln wie Essigester oder Dichlormethan.

Der 3-Methylamino-crotonsäuremethylester läßt sich beispielsweise aus Methylamin und Acetessigsäuremethylester herstellen. Die metasubstituierten Benzoylhalogenide, die zur Acylierung dieses Enamins verwendet werden, werden aus den entsprechenden Benzoesäuren und Thionylchlorid bzw. Thionylbromid erhalten. Die jeweils verwendeten Benzoesäuren werden nach im Prinzip bekannten Verfahren hergestellt, z.B. 3-Benzyloxy-benzoesäure durch Umsetzung von 3-Hydroxybenzoesäure-dinatriumsalz mit Benzylchlorid, oder 3-Phenoxymethyl-benzoesäure durch Umsetzung von 3-Cyano-benzylchlorid mit Natriumphenolat und anschließende alkalische Verseifung der Nitrilgruppe in KOH/Ethylenglykol.

Die folgenden Beispiele erläutern die Herstellung der Pyrazolderivate der Formel I.

Beispiel 1

5-Methyl-4-methoxycarbonyl-3-(3-benzyloxy-phenyl)-pyrazole

Zu einer Lösung von 64,5 g 3-Methylaminocrotonsäuremethylester in 150 ml Toluol gibt man 47 g Pyridin und kühlt auf 0°C ab. Anschließend tropft man 123,3 g 3-Benzyloxy-benzoylchlorid so zu, daß die Reaktionstemperatur under 5°C bleibt. Nach 16 stündigem Nachrühren wird das Pyridinhydrochlorid durch Absaugen entfernt, das Filtrat wird mit Wasser extrahiert. Nach Trocknen der Toluollösung wird unter vermindertem Druck eingedampft. Man erhält 145 g eines Öls (=85% d.Th.).

Das so erhaltene Öl wird in 200 ml Eisessig gelöst und danach tropfenweise bei 25 bis 30°C mit 50 g Hydrazinhydrat versetzt. Anschließend läßt man das Reaktionsgemisch 3 Stunden refluxieren, versetzt anschließend mit 300 ml Wasser und extrahiert mit 3 × 250 ml Diethylether. Die Etherphasen werden vereint und über Magnesiumsulfat getrocknet. Danach leitet man zur Sättigung HCl-Gas ein, so daß Pyrazolderivat als Hydrochlorid ausfällt. Das durch Absaugen erhaltene Hydrochlorid wird in 200 ml Essigester gegeben und mit 25 %igem $NH_3$-Wasser versetzt, bis die wäßrige Phase deutlich alkalisch bleibt. Nach Abtrennen der organischen Phase verbleiben nach Trocknen und Einengen 81 g 5-Methyl-4-methoxycarbonyl-3-(3-benzyloxyphenyl)-pyrazol (58% d.Th.) in Form eines Öls.

Beispiel 2

5-Methyl-4-methoxycarbonyl-3-(3-benzyloxy-phenyl)-1-acetyl-pyrazol

Durch Kochen von 5-Methyl-4-methoxycarbonyl-3-(3-benzyloxyphenyl)-pyrazol mit überschüssigem Acetanhydrid im Molverhältnis 1:4 erhält man 5-Methyl-4-methoxycarbonyl-3-(3-benzyloxyphenyl)-1-acetyl-pyrazol, das ebenfalls in Form eines Öls anfällt.

Beispiel 3

5-Methyl-4-methoxycarbonyl-3-[3-(2-(2-chlorphenoxy)-ethoxy)-phenyl]-pyrazol

In analoger Weise wie in Beispiel 1 erhält man aus 3-Methylamino-crotonsäuremethylester (0,5 Mol) und 3-[2-(2-Chlorphenoxy)-ethoxy]-benzoylchlorid (0,5 Mol) 3-Methylamino-2-[3-(2-(2-chlor-phenoxy)-ethoxy)-benzoyl]-crotonsäuremethylester vom Fp. 131 bis 133°C in 58% Ausbeute. Dieser Ester liefert bei der Umsetzung mit Hydrazinhydrat analog Beispiel 1 5-Methyl-4-methoxycarbonyl-3-[3-(2-(2-chlorphenoxy)-ethoxy)-phenyl]-pyrazol vom Fp. 127 bis 129°C in 59% Ausbeute.

In entsprechender Weise können folgende Pyrazolderivate der Formel I erhalten werden:

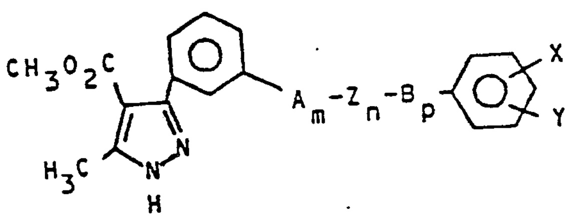

| Verbindung Nr. | m | n | p | A | Z | B | X | Y | Fp. [°C] |
|---|---|---|---|---|---|---|---|---|---|
| 1 | Null | Null | 1 | - | - | O | H | H | amorph |
| 2 | Null | Null | 1 | - | - | O | 2-Cl | H | Öl |
| 3 | Null | Null | 1 | - | - | O | 3-Cl | H | Öl |
| 4 | Null | Null | 1 | - | - | O | H | 4-Br | 129-130 |
| 5 | Null | Null | 1 | - | - | O | 2-Cl | 4-$CF_3$ | amorph |
| 6 | Null | Null | 1 | - | - | O | 2-$NO_2$ | 4-$CF_3$ | 85- 87 |
| 7 | Null | Null | 1 | - | - | O | 2-Cl | 4-$NO_2$ | 115-117 |
| 8 | Null | Null | 1 | - | - | O | H | 4-$NO_2$ | 103 |
| 9 | 1 | 1 | Null | O | -$CH_2$- | - | H | H | Öl |

| Verbindung Nr. | m | n | p | A | Z | B | X | Y | Fp. [°C] |
|---|---|---|---|---|---|---|---|---|---|
| 10 | 1 | 1 | Null | O | $-CH_2-$ | - | H | $3-CH_3$ | Öl |
| 11 | 1 | 1 | Null | O | $-CH_2-$ | - | H | $4-CH_3$ | 115-117 |
| 12 | 1 | 1 | Null | O | $-CH_2-$ | - | H | 2-F | Öl |
| 13 | 1 | 1 | Null | O | $-CH_2-$ | - | H | 4-F | Öl |
| 14 | 1 | 1 | Null | O | $-CH_2-$ | - | H | $3-CF_3$ | Öl |
| 15 | 1 | 1 | Null | O | $-CH_2-$ | - | H | $3-OCH_3$ | Öl |
| 16 | 1 | 1 | Null | O | $-CH_2-$ | - | H | 3-Cl | 126-128 |
| 17 | 1 | 1 | Null | O | $-CH_2-$ | - | 4-Cl | H | 139-141 |
| 18 | 1 | 1 | Null | O | $-CH_2-$ | - | 2-Cl | 6-Cl | 139-142 |
| 19 | 1 | 1 | Null | O | $-CH_2-$ | - | 2-Cl | 4-Cl | 167-169 |
| 20 | 1 | 1 | Null | O | $-CH_2-$ | - | 3-Cl | $4-CF_3$ | 58 |
| 21 | 1 | 1 | 1 | O | $-CH_2CH_2-$ | O | H | H | 134-136 |
| 22 | 1 | 1 | 1 | O | $-CH_2CH_2-$ | O | H | $2-CH_3$ | 126-128 |
| 23 | 1 | 1 | 1 | O | $-CH_2CH_2-$ | O | H | $4-CH_3$ | 191-193 |
| 24 | 1 | 1 | 1 | O | $-CH_2CH_2-$ | O | 2-Cl | H | 127-129 |
| 25 | 1 | 1 | 1 | O | $-CH_2CH_2-$ | O | 4-Cl | H | 188-190 |
| 26 | 1 | 1 | 1 | O | $-CH_2CH_2CH_2-$ | O | H | H | Öl |
| 27 | 1 | 1 | 1 | O | $-(CH_2)_4-$ | O | H | H | 86-88 |
| 28 | Null | 1 | 1 | - | $-CH_2-$ | O | H | 3-Cl | 48-50 |
| 29 | Null | 1 | 1 | - | $-CH_2-$ | O | H | 4-Cl | Öl |
| 30 | 1 | 1 | Null | S | $-CH_2-$ | - | H | 4-Cl | |

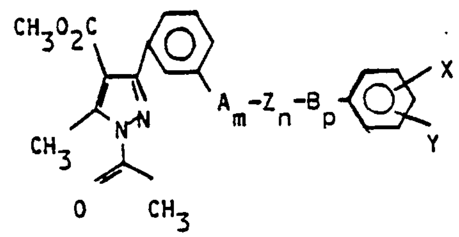

| Verbindung Nr. | m | n | p | A | Z | B | X | Y | Fp. [°C] |
|---|---|---|---|---|---|---|---|---|---|
| 31 | 1 | Null | Null | O | - | - | H | 4-Br | 114-116 |
| 32 | 1 | Null | Null | O | - | - | 2-Cl | $4-CF_3$ | 115-117 |
| 33 | 1 | Null | Null | O | - | - | $2-NO_2$ | $4-CF_3$ | 151-153 |
| 34 | 1 | 1 | Null | O | $-CH_2-$ | - | H | H | Öl |
| 35 | 1 | 1 | Null | O | $-CH_2-$ | - | H | $3-CH_3$ | Öl |
| 36 | 1 | 1 | Null | O | $-CH_2-$ | - | H | $4-CH_3$ | 110-112 |

| Verbindung Nr. | m | n | p | A | Z | B | X | Y | Fp. [°C] |
|---|---|---|---|---|---|---|---|---|---|
| 37 | 1 | 1 | Null | O | $-CH_2-$ | - | H | 2-F | Öl |
| 38 | 1 | 1 | Null | O | $-CH_2-$ | - | H | 4-F | Öl |
| 39 | 1 | 1 | 1 | O | $-CH_2CH_2-$ | O | H | H | 117-119 |
| 40 | 1 | 1 | 1 | O | $-CH_2CH_2-$ | O | H | $2-CH_3$ | 117 |
| 41 | 1 | 1 | 1 | O | $-CH_2CH_2-$ | O | 2-Cl | H | 96- 98 |
| 42 | 1 | 1 | 1 | O | $-(CH_2)_4-$ | O | H | H | 90- 93 |
| 43 | 1 | 1 | 1 | O | $-(CH_2)_5-$ | O | H | H | 52- 55 |
| 67 | Null | 1 | 1 | - | $-CH_2-$ | O | 2-Cl | 3-Cl | |
| 68 | Null | 1 | 1 | - | $-CH_2-$ | O | 3-Cl | 5-Cl | |
| 69 | Null | 1 | 1 | - | $-CH_2-$ | O | 3-F | H | |
| 70 | Null | 1 | 1 | - | $-CH_2-$ | O | 3-Cl | H | |

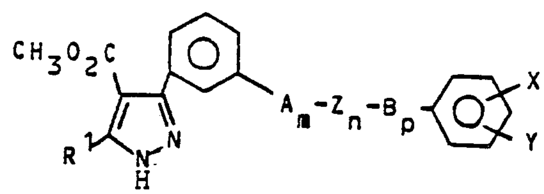

| Verbindung Nr. | $R^1$ | m | n | p | A | Z | B | X | Y | Fp. [°C] |
|---|---|---|---|---|---|---|---|---|---|---|
| 44 | $CH_3$ | 1 | 1 | Null | O | $-CH_2-$ | - | H | 3-F | |
| 45 | $CH_3$ | 1 | 1 | Null | O | $-CH_2-$ | - | H | 3-CN | |
| 46 | $CH_3$ | Null | 1 | 1 | - | $-CH_2-$ | O | $4-CF_3$ | H | |
| 47 | $CH_3$ | Null | 1 | 1 | - | $-CH_2-$ | O | H | $3-CF_3$ | Öl |
| 48 | $CH_3$ | Null | 1 | 1 | - | $-CH_2-$ | O | 3-F | H | 108-110 |
| 49 | $CH_3$ | Null | 1 | 1 | - | $-CH_2-$ | O | 2-Cl | 3-Cl | Öl |
| 50 | $CH_3$ | Null | 1 | 1 | - | $-CH_2-$ | O | 2-Cl | 4-Cl | 120-122 |
| 51 | $CH_3$ | Null | 1 | 1 | - | $-CH_2-$ | O | 3-Cl | 4-Cl | 105-107 |
| 52 | $CH_3$ | Null | 1 | 1 | - | $-CH_2-$ | O | 2-Cl | 5-Cl | |
| 53 | $CH_3$ | Null | 1 | 1 | - | $-CH_2-$ | O | 3-Cl | 5-Cl | Öl |
| 54 | $CH_3$ | 1 | 1 | Null | S | $-CH_2CH_2-$ | - | H | H | |
| 55 | $CH_3$ | 1 | 1 | Null | S | $-CH_2CH_2CH_2-$ | - | H | H | |
| 56 | $CH_3$ | 1 | 1 | 1 | S | $-CH_2CH_2-$ | O | 3-Cl | H | |
| 57 | $CH_3$ | 1 | 1 | 1 | S | $-CH_2CH_2-$ | S | H | H | |
| 58 | $CH_3$ | 1 | 1 | 1 | O | $-CH_2CH_2-$ | S | H | H | |
| 59 | $CH_3$ | 1 | 1 | 1 | O | $-CH_2CH_2-$ | S | 4-Cl | H | |

| Verbindung Nr. | $R^1$ | m | n | p | A | Z | B | X | Y | Fp. [°C] |
|---|---|---|---|---|---|---|---|---|---|---|
| 60 | $CH_3$ | Null | 1 | Null | - | $-(CH_2)_3-$ | - | H | H | |
| 61 | $CH_3$ | Null | 1 | Null | - | $-(CH_2)_4-$ | - | H | H | |
| 62 | $CH_3$ | Null | 1 | Null | - | $-(CH_2)_5-$ | - | H | H | |
| 63 | $CH_3$ | 1 | 1 | Null | S | $-CH_2-CH(CH_3)-CH_2-$ | - | H | H | |
| 64 | H | 1 | Null | Null | O | - | - | H | H | |
| 65 | H | 1 | Null | Null | O | - | - | 3-Cl | H | |
| 66 | H | Null | 1 | 1 | - | $-CH_2-$ | O | 3-Cl | H | |

Die Pyrazolderivate der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Natphalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoetheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt, werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoff, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gew.-Teile der Verbindung Nr. 13 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 16 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzosulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile der Verbindung Nr. 17 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

IV. 20 Gew.-Teile des Wirkstoffs Nr. 12 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

V. 20 Gew.-Teile des Wirkstoffs Nr. 14 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigen Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

VI. 3 Gew.-Teile des Wirkstoffs Nr. 38 werden mit 97 Gew.-Teilen feinteiligem Kaolin vermischt. Man erhält auf diese Weise ein Stäubemittel, das 3 Gew.% des Wirkstoffs enthält.

VII. 30 Gew.-Teile des Wirkstoffs Nr. 31 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 9 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile öbige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,05 bis 5,0, vorzugsweise 0,1 bis 3,0 kg/ha.

Die herbizide Wirkung der 3-Phenyl-4-methoxycarbonylpyrazole der Formel I auf das Wachstum von unerwünschten Pflanzen wird durch folgende Gewächshausversuche gezeigt.

Als Kulturgefäße dienen Plastikblumentöpfe mit 300 $cm^3$ Inhalt und lehmigem Sand mit etwa 1,5% Humus als Substrat. Bei Soja wird etwas Torfmull zugesetzt, um einen besseren Stand zu erzielen. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff; sie betragen beispielsweise 0,25, 0,5, 1,0 und 2,0 kg Wirkstoff/ha. Eine Abdeckung unterbleibt bei der Nachflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dier Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumidest der oberirdischen Teile.

Die in den Gewächshausversuchen verwendeten Pflanzen setzen sich aus folgenden Arten zusammen:

Amaranthus retroflexus (zurückgekrümmter Fuchsschwanz), Amaranthus spp. (Fuchsschwanzarten), Avena sativa (Hafer), Chenopodium album (Weißer Gänsefuß), Cassia tora, Echinochloa crus-galli (Hühnerhirse), Galeopsis tetrahit (Gemeiner Hohlzahn), Galium aparine (Klettenlabkraut), Glycine max (Soja), Gossypium hirsutum (Baumwolle), Ipomoea spp. (Prunkwindearten), Sinapis alba (Weißer Senf), Solanum nigrum (Schwarzer Nachtschatten), Triticum aestivum (Weizen), Arachys hypogaea (Erdnüsse), Ipomoea spp. (Prunkwindearten), Sida spinosa, Lamium amplexicaule (Stengelumfassende Taubnessel), Helanthus annuus (Sonnenblume).

Vorauflaufanwendung

Bei einer Aufwandmenge von 3,0 kg Wirkstoff/ha wirken beispielsweise die Verbindungen Nr. 5, 13 und 26 gegen die breitblättrige Testpflanze Sinapis alba, ohne Kulturhafer zu schädigen.

Nachauflaufanwendung

Bei einer Aufwandmenge von 3,0 kg Wirkstoff/ha bekämpfen beispielsweise die Verbindungen Nr. 9, 12, 13, 14, 15, 16, 18, 38, 37, 39, 41 und 34 gegen eine Reihe unerwünschter breitblättriger Pflanzen. Dasselbe gilt für die Verbindungen Nr. 13, 16, 17, 36 und 38 bei einer Aufwandmenge von 1,0 kg/ha. Echinochloa crus-galli wird mit 3,0 kg/ha durch die Verbindungen Nr. 1, 2, 3, 8 und 17 bekämpft. Geeignet zur selektiven Bekämpfung von unerwünschten breitblättrigen Pflanzen in Weizen sind die Verbindungen Nr. 5, 31 und 17 mit 0,5 kg Wirkstoff/ha sowie die Verbindungen Nr. 12, 14 und 38 mit 0,25 kg Wirkstoff/ha. Die Verbindung Nr. 38 bekämpft mit 0,25 kg Wirkstoff/ha unerwünschte breitblättrige Pflanzen in Sojabohnen, wobei diese anfänglich noch geringfügig geschädigt werden. Dasselbe gilt für Verbindung Nr. 9 bei einer Aufwandmenge von 1,0 kg Wirkstoff/ha in Baumwolle.

Mit den Verbindungen Nr. 28, 53 und 67 werden beispielsweise bei einer Aufwandmenge von 0,5 kg Wirkstoff/ha ebenfalls breitblättrige Unkraüter erfaßt, während Sonnenblumen nur temporär und leicht geschädigt werden. Ebenso ziegen die Verbindungen Nr. 26 und 29 mit 0,5 kg Wirkstoff/ha eine beachtliche herbizide Aktivität gegen unerwünschte Pflanzen bei nur leichten Schäden an Erdnüssen als beispielhaft ausgewählt Kultur.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Brassica napus var. rapa | Weiße Rübe |
| Camellia sinensis | Teestrauch |
| Carthamus tinctorius | Saflor — Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cynodon dactylon | Bermudagras |
| Daucus carota | Möhre |
| Elaeis guineensis | Ölpalme |
| Fragaria vesca | Erdbeere |
| Glyoine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |

| Botanischer Name | Deutscher Name |
|---|---|
| Medicago sativa | Luzerne |
| Mentha piperita | Pfefferminze |
| Musa spp. | Obst- und Mehlbanane |
| Nicotiana tabacum (N-rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Waißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |
| Theobroma cacao | Kakaobaum |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 3-Phenyl-4-methoxycarbonylpyrazole der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Pyrazolderivate der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. 3-Phenyl-4-methoxycarbonyl-pyrazolderivate der Formel

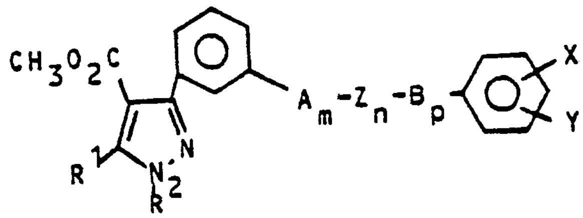

(I),

in der

$R^1$ für Wasserstoff oder Methyl,

$R^2$ für Wasserstoff, gegebenenfalls durch Halogen substituiertes $C_1$—$C_4$-Alkanoyl, Methoxycarbonyl, gegebenenfalls durch Halogen oder $C_1$—$C_4$-Alkyl substituiertes Phenoxycarbonyl, für Hydroxymethyl oder Methoxymethyl,

A und B unabhängig voneinander für Sauerstoff oder Schwefel,

Z für eine gegebenenfalls durch Methyl substituierte Alkylenkette mit 1 bis 5 Kohlenstoffatomen,

m, n und p unabhängig voneinander für Null oder 1,

X für Wasserstoff, Halogen oder Nitro und

Y für Wasserstoff, $C_1$—$C_4$-Alkyl, $C_1$—$C_4$-Alkoxy, $C_1$—$C_4$-Halogenalkyl, Halogen, Nitro oder Cyano stehen.

2. 3-Phenyl-4-methoxycarbonyl-pyrazolderivate der Formel I gemäß Anspruch 1, dadurch gekennzeichnet, daß $R^1$ Methyl bedeuten.

3. 3-Phenyl-4-methoxycarbonyl-pyrazolderivate der Formel I gemäß Anspruch 1, dadurch gekennzeichnet, daß $R^2$ Wasserstoff oder Acetyl bedeutet.

4. 5-Methyl-4-methoxycarbonyl-3-[3-(3-chlor-benzyloxy)-phenyl]-pyrazol.

5. 5-Methyl-4-methoxycarbonyl-3-(3-benzyloxy-phenyl)-1-acetyl-pyrazol.

6. Verfahren zur Herstellung von 3-Phenyl-4-methoxycarbonyl-pyrazol-derivaten der Formel I gemäß Anspruch 1, dadurch gekennzeichnet, daß man 3-Alkylaminopropensäuremethylester der Formel

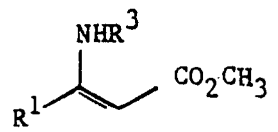

(II),

in der

$R^1$ für Wasserstoff oder Methyl und $R^3$ für $C_1$—$C_4$-Alkyl stehen, mit Benzoesäurehalogeniden der Formel

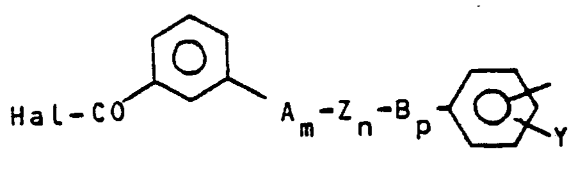

(III),

in der

Hal für Halogen steht und A, B, Z, X, Y, m, n und p die in Anspruch 1 genannten Bedeutungen haben, umsetzt und die so erhaltenen 2-Benzoyl-propensäuremethylester mit mindestens der äquimolaren Menge Hydrazinhydrat umsetzt und die so erhaltenen Pyrazolderivate gegebenenfalls in an sich bekannter Weise acyliert, hydroxymethyliert oder methoxymethyliert.

7. Herbizid, enthaltend inerte Zusatzstoffe und ein 3-Phenyl-4-methoxycarbonyl-pyrazolderivate der Formel

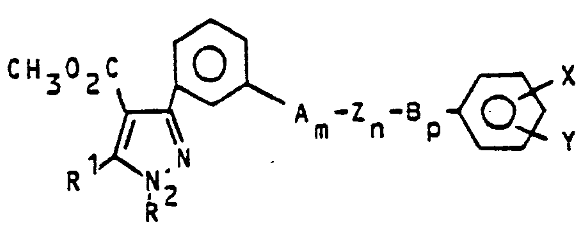

(I),

in der

$R^1$ für Wasserstoff oder Methyl,

$R^2$ für Wasserstoff, gegebenenfalls durch Halogen substituiertes $C_1$—$C_4$-Alkanoyl, Methoxycarbonyl,

gegebenenfalls durch Halogen oder $C_1$—$C_4$-Alkyl substituiertes Phenoxycarbonyl, für Hydroxymethyl oder Methoxymethyl,

A und B unabhängig voneinander für Sauerstoff oder Schwefel,

Z für eine gegebenenfalls durch Methyl substituierte Alkylenkette mit 1 bis 5 Kohlenstoffatomen,

m, n und p unabhängig voneinander für Null oder 1,

X für Wasserstoff, Halogen oder Nitro und

Y für Wasserstoff, $C_1$—$C_4$-Alkyl, $C_1$—$C_4$-Alkoxy, $C_1$—$C_4$-Halogenalkyl, Halogen, Nitro oder Cyano stehen.

8. Herbizid nach Anspruch 7, dadurch gekennzeichnet, daß es ein 3-Phenyl-4-methoxycarbonyl-pyrazolderivat der Formel I enthält, wobei $R^1$ Methyl bedeutet.

9. Herbizid nach Anspruch 7, dadurch gekennzeichnet, daß es ein 3-Phenyl-4-methoxycarbonyl-pyrazolderivat der Formel I enthält, wobei $R^2$ Wasserstoff oder Acetyl bedeutet.

10. Verfahren zur Bekämpfung unerwünschten Pflanzenwuchses, dadurch gekennzeichnet, daß man die Pflanzen und/oder ihren Standort mit einer herbizid wirksamen Menge eines 3-Phenyl-4-methoxycarbonyl-pyrazolderivats der Formel I gemäß Anspruch 1 behandelt.

**Revendications**

1. Dérivé de 3-phényl-4-méthoxycarbonyl-pyrazole de formule

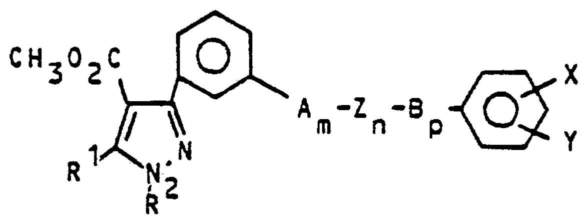

(I),

dans laquelle

$R^1$ est mis pour hydrogène, ou méthyle,

$R^2$ pour hydrogène, alcanoyle en $C_1$—$C_4$, éventuellement substitué par halogène, méthoxycarbonyle, phénoxycarbonyle éventuellement substitué par halogène ou alkyle en $C_1$—$C_4$, pour hydroxyméthyle ou méthoxyméthyle,

A et B, indépendamment l'un de l'autre, pour oxygène ou soufre,

Z, pour une chaîne alkylène de 1 à 5 atomes de carbone, éventuellement substituée par méthyle,

m, n et p, indépendamment l'un de l'autre, pour 0 ou 1,

X, pour hydrogène, halogène ou nitro et

Y, pour hydrogène, alkyle en $C_1$—$C_4$, alcoxy en $C_1$—$C_4$, halogénalkyle en $C_1$—$C_4$, halogène, nitro ou cyano.

2. Dérivé de 3-phényl-4-méthoxycarbonyl-pyrazole de formule I selon la revendication 1, caractérisé par le fait que $R^1$ est mis pour méthyle.

3. Dérivé de 3-phényl-4-méthoxycarbonyl-pyrazole de formule I selon la revendication 1, caractérisé par le fait que $R^2$ représente hydrogène.

4. 5-méthyl-4-méthoxycarbonyl-3-[3-(3-chloro-benzyloxy)-phényl]-pyrazole.

5. 5-méthyl-4-méthoxycarbonyl-3-(3-benzyloxy-phényl)-1-acétyl-pyrazole.

6. Procédé de préparation de dérivé de 3-phényl-4-méthoxycarbonyl-pyrazole de formule I selon la revendication 1, caractérisé par le fait que l'on fait réagir du 3-alkylamino-propénate de méthyle de formule

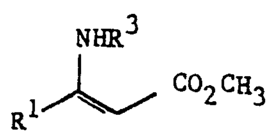

(II),

dans laquelle

$R^1$ représente hydrogène ou méthyle et

$R^3$, alkyle en $C_1$—$C_4$, avec de l'halogénure d'acide benzoïque de formule

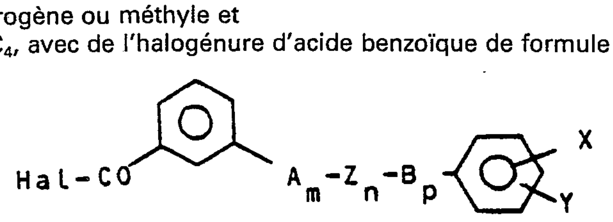

(III),

dans laquelle

Hal est mis pour halogène et A, B, Z, X, Y, m, n et p ont les significations données dans la revendication

1, et on fait réagir le 2-benzoylpropénate de méthyle ainsi obtenu avec au moins la quantité équimolaire d'hydrate d'hydrazine, et on acyle, hydroxyméthyle ou méthoxyméthyle, de manière connue en soi, éventuellement, le dérivé de pyrazole ainsi obtenu.

7. Herbicide contenant des additifs inertes et un dérivé de 3-phényl-4-méthoxycarbonyl-pyrazole de formule

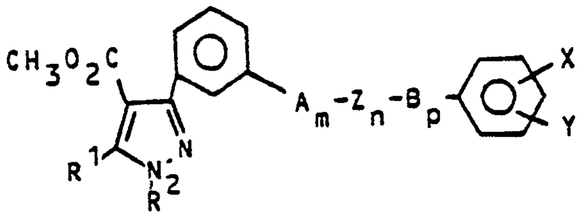

(I),

dans laquelle

$R^1$ est mis pour hydrogène, ou méthyle,

$R^2$ pour hydrogène, alcanoyle en $C_1$—$C_4$, éventuellement substitué par halogène, méthoxycarbonyle, phénoxycarbonyle éventuellement substitué par halogène ou alkyle en $C_1$—$C_4$, pour hydroxyméthyle ou méthoxyméthyle,

A et B, indépendamment l'un de l'autre, pour oxygène ou soufre,

Z, pour une chaîne alkylène de 1 à 5 atomes de carbone, éventuellement substituée par méthyle,

m, n et p, indépendamment l'un de l'autre, pour 0 ou 1,

X, pour hydrogène, halogène ou nitro et

Y, pour hydrogène, alkyle en $C_1$—$C_4$, alcoxy en $C_1$—$C_4$, halogénalkyle en $C_1$—$C_4$, halogène, nitro ou cyano.

8. Herbicide selon la revendication 7, caractérisé par le fait qu'il contient un dérivé de 3-phényl-4-méthoxy-carbonyl-pyrazole de formule I où $R^1$ représente méthyle.

9. Herbicide selon la revendication 7, caractérisé par le fait qu'il contient un dérivé de 3-phényl-4-méthoxycarbonyl-pyrazole de formule I où $R^2$ représente hydrogène ou acétyle.

10. Procédé de lutte contre la croissance de plantes indésirables, caractérisé par le fait que l'on traite les plantes et/ou leur biotope avec une quantité efficace au point de vue herbicide d'un dérivé de 3-phényl-4-méthoxycarbonyl-pyrazole de formule I selon la revendication 1.

**Claims**

1. A 3-phenyl-4-methoxycarbonylpyrazole derivative of the formula

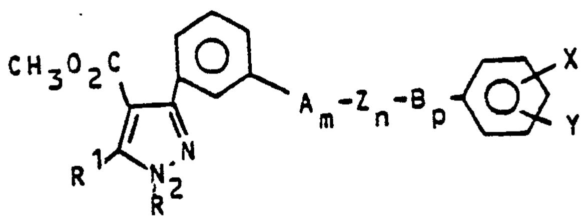

(I),

where $R^1$ is hydrogen or methyl, $R^2$ is hydrogen, unsubstituted or halogen-substituted $C_1$—$C_4$-alkanoyl, methoxycarbonyl, or phenoxycarbonyl which is unsubstituted or substituted by halogen or $C_1$—$C_4$-alkyl, or is hydroxymethyl or methoxymethyl, A and B independently of one another are each oxygen or sulfur, Z is unsubstituted or methyl-substituted alkylene of 1 to 5 carbon atoms, m, n and p independently of one another are each zero of 1, X is hydrogen, halogen or nitro, and Y is hydrogen, $C_1$—$C_4$-alkyl, $C_1$—$C_4$-alkoxy, $C_1$—$C_4$-haloalkyl, halogen, nitro or cyano.

2. A 3-phenyl-4-methoxycarbonylpyrazole derivative of the formula I as claimed in claim 1, where $R^1$ is methyl.

3. A 3-phenyl-4-methoxycarbonylpyrazole derivative of the formula I as claimed in claim 1, where $R^2$ is hydrogen or acetyl.

4. 5-Methyl-4-methoxycarbonyl-3-[3-(3-chlorobenzyloxy)-phenyl]-pyrazole.

5. 5-Methyl-4-methoxycarbonyl-3-(3-benzyloxyphenyl)-1-acetylpyrazole.

6. A process for the preparation of 3-phenyl-4-methoxycarbonylpyrazole derivatives of the formula I as claimed in claim 1, wherein a methyl 3-alkylamino-propenoate of the formula

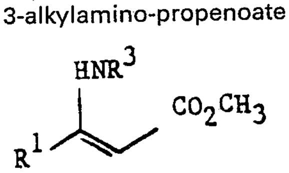

(II),

where $R^1$ is hydrogen or methyl and $R^3$ is $C_1$—$C_4$-alkyl, is reacted with a benzoyl halide of the formula

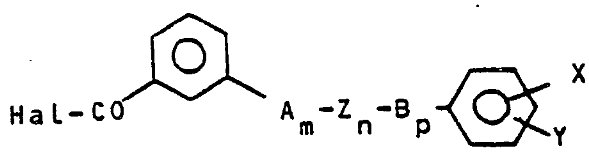
(III),

where Hal is halogen and A, B, Z, X, Y, m, n and p have the meanings given in claim 1, and the methyl 2-benzoyl-propenoate which is obtained is reacted with at least an equimolar amount of hyrazine hydrate, and the pyrazole derivative which is obtained is if desired acylated, hydroxymethylated or methoxymethylated in a conventional manner.

7. A herbicide containing inert additives and a 3-phenyl-4-methoxycarbonylpyrazole derivative of the formula

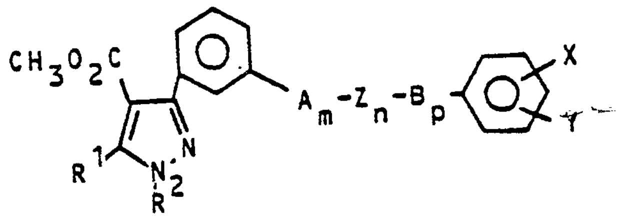
(I),

where $R^1$ is hydrogen or methyl, $R^2$ is hydrogen, unsubstituted or halogen-substituted $C_1$—$C_4$-alkanoyl, methoxycarbonyl, or phenoxycarbonyl which is unsubstituted or substituted by halogen or $C_1$—$C_4$-alkyl, or is hydroxymethyl or methoxymethyl, A and B independently of one another are each oxygen or sulfur, Z is unsubstituted or methyl-substituted alkylene of 1 to 5 carbon atoms, m, n and p independently of one another are each zero or 1, X is hydrogen, halogen or nitro, and Y is hydrogen, $C_1$—$C_4$-alkyl, $C_1$—$C_4$-alkoxy, $C_1$—$C_4$-haloalkyl, halogen, nitro or cyano.

8. A herbicide as claimed in claim 7, which contains a 3-phenyl-4-methoxycarbonylpyrazole derivative of the formula I, where $R^1$ is methyl.

9. A herbicide as claimed in claim 7, which contains a 3-phenyl-4-methoxycarbonylpyrazole derivative of the formula I, where $R^2$ is hydrogen or acetyl.

10. A method for controlling undesirable plant growth, wherein the plants and/or their location are or is treated with a herbicidally effective amount of a 3-phenyl-4-methoxycarbonylpyrazole derivative of the formula I as claimed in claim 1.